(19) Europäisches Patentamt
European Patent Office
Office européen des brevets

(11) Veröffentlichungsnummer: **0 408 881 A2**

(12) # EUROPÄISCHE PATENTANMELDUNG

(21) Anmeldenummer: **90110686.4**

(22) Anmeldetag: **06.06.90**

(51) Int. Cl.5: **C07C 227/32**, C07C 229/22, C07C 229/36, C07C 271/22

Die Bezeichnung der Erfindung wurde geändert (Richtlinien für die Prüfung im EPA, A-III, 7.3).

(30) Priorität: **19.06.89 DE 3919898**

(43) Veröffentlichungstag der Anmeldung: **23.01.91 Patentblatt 91/04**

(84) Benannte Vertragsstaaten: **AT BE CH DE DK ES FR GB IT LI NL SE**

(71) Anmelder: **MERCK PATENT GESELLSCHAFT MIT BESCHRÄNKTER HAFTUNG**
**Frankfurter Strasse 250 Postfach 4119**
**D-6100 Darmstadt(DE)**

(72) Erfinder: **Radunz, Hans-Eckart, Dr.**
**Am Klingenteich 5**
**D-6109 Mühltal(DE)**
Erfinder: **Reissig, Hans Ulrich, Prof. Dr.**
**Heinrich-Rinck-Weg 2**
**D-6100 Darmstadt(DE)**

(54) **Verfahren zur enantioselektiven Herstellung von gamma-Keto-delta-aminosäurederivaten.**

(57) Die Erfindung betrifft ein Verfahren zur enantioselektiven Herstellung von γ-Keto-δ-Aminosäurederivaten der Formel I,

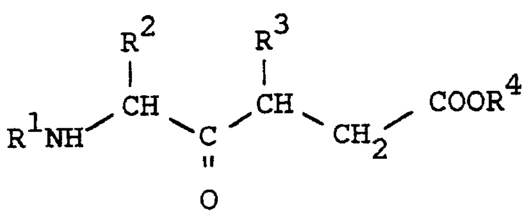

I

dadurch gekennzeichnet, daß man eine Verbindung der Formel II

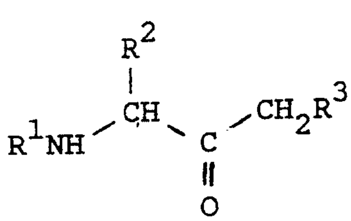

II

mit einem Trialkylchlorsilan in eine Verbindung der Formel III

EP 0 408 881 A2

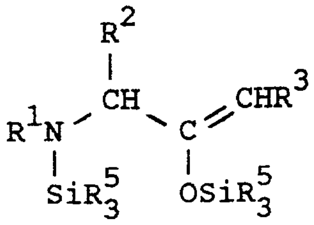

III

überführt, dann diese Verbindung mit einem Diazoessigester der Formel $N_2CH_2COOR^4$ ($R^4 \neq H$) zu einer Cyclopropanverbindung der Formel IV

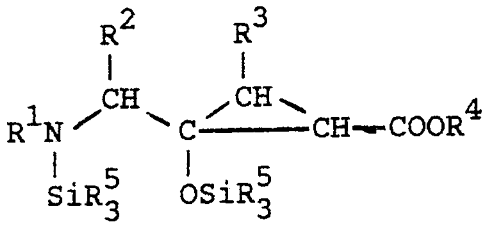

IV

umsetzt, und anschließend die Verbindung IV durch Ringöffnung und Abspaltung der Silylgruppen sowie gegebenenfalls Verseifung in die Verbindung der Formel I überführt, wobei $R^1$, $R^2$, $R^3$, $R^4$ und $R^5$ die im Anspruch 1 angegebenen Bedeutungen haben.

## VERFAHREN ZUR ENANTIOSELEKTIVEN HERSTELLUNG VON γ-KETO-δ-AMINOSÄUREDERIVATEN

Die Erfindung betrifft ein Verfahren zur enantioselektiven herstellung von γ-Keto-δ-Aminosäurederivaten der Formel I

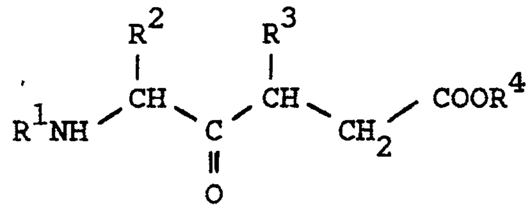

I

worin

$R^1$ eine Aminoschutzgruppe oder H,

$R^2$ eine Alkylgruppe mit 1 bis 5 C-Atomen, die gegebenenfalls ein- oder zweifach substituiert sein kann durch -OH, -COOH, $-CONH_2$ oder $-NH_2$, oder worin gegebenenfalls eine $CH_2$-Gruppe durch -S- ersetzt sein kann, unsubstituiertes oder durch 1 bis 5 C-Atome enthaltendes Alkyl oder Alkoxy, Halogen, Hydroxy oder Amino ein- oder mehrfach substituiertes Phenyl oder Cyclohexyl, Phenylalkyl oder Cyclohexylalkyl oder Het-alkyl-, wobei

Het einen gesättigten oder ungesättigten 5-oder 6-gliedrigen heterocyclischen Rest mit 1-4 N-Atomen, der mit einem Benzolring kondensiert sein kann, und

-alkyl- eine Alkylengruppe mit 1-5 C-Atomen darstellt,

und

$R^3$ und $R^4$ jeweils unabhängig voneinander H oder Alkyl mit 1-5 C-Atomen

bedeuten.

Verbindungen der Formel I werden als Ketomethylenanaloge eines Dipeptids eingesetzt und finden Verwendung als Baustein in Inhibitoren des Angiotensin Converting Enzymes, beschrieben von z.B. R.G. Almquist et al. in J. Med. Chem. 31 , 561 (1988), oder in Tachykininanalogen, beschrieben von A. Ewenson et al. in J. Med. Chem. 29 , 295 1986.

Der Einbau dieser Aminosäuren der Formel I und deren reduzierte Formen (γ-Hydroxy-δ-Aminosäuren) ist auch in anderen pharmakologisch interessanten Peptiden, wie z.B. Inhibitoren der Aspartylproteasen, möglich.

Die enantioselektive Herstellung von γ-Keto-δ-Aminosäuren ist bislang nur ungenügend möglich. Beispielsweise ist die Synthese eines optisch aktiven Ketomethylenanalogen des Dipeptids L-Phenylalanylglycin von R.G. Almquist in J.Med.Chem. 23 , 1392 (1980) beschrieben. Diese Synthese verläuft über 6 Stufen, wobei zunächst ein N-Phthaloyl-L-phenylalanin-2-pyridyl-thioester mit 2-(2-Magnesium-2-bromethyl)-1,3-dioxolan zum entsprechenden Ketoacetal (vgl. M. Araki et al., Bull.Chem.Soc.Jpn., 47 , 1777 (1974) und K. Lloyd et al., J.Chem.Soc.C, 2890 (1971)) umgesetzt wird. Die Ketogruppe wird dann analog W.S. Johnson et al., J.Am.Chem. Soc., 78 , 6289 (1956) in das Ethylenketal überführt, anschließend wird die Phthaloylgruppe einer Hydra zinolyse unterworfen, gefolgt von einer Benzoylierung des vorher entstandenen Amins. Dann wird im 5. und 6. Schritt der Synthese zuerst zur intermediären Ketalsäure oxidiert und dann die Ketalgruppe mit Trifluoressigsäure entfernt.

Eine andere Synthese ist beschrieben von A. Ewenson et al. in J.Med.Chem. 29 295 (1986), bei welcher jedoch ein Enantiomerengemisch entsteht.

Da für die Wirksamkeit eines solchen Produktes die (S)-Konfiguration am C-Atom, das die $-NHR^1$-Gruppe trägt, bevorzugt ist, ist eine enantioselektive Synthese notwendig.

Die bisher bekannten Verfahren haben den Nachteil, daß eine Enantioselektivität nicht gegeben ist oder eine Vielzahl von aufwendigen Syntheseschritten notwendig ist.

Es bestand also die Aufgabe, ein Verfahren zu finden, das die Herstellung von wertvollen Verbindungen der Formel I mit sehr hoher Enantioselektivität und guter Ausbeute auf einfache und wirtschaftliche Weise ermöglicht.

Überraschend wurde nun gefunden, daß durch Umsetzung eines entsprechenden Aminoketons über einen Silylenolether zum entsprechenden Cyclopropanderivat und anschließende Ringöffnung Ketoaminosäuren der Formel I enantioselektiv erhalten werden können.

Gegenstand der Erfindung ist somit ein Verfahren zur enantioselektiven Herstellung von γ-Keto-δ-Aminosäurederivaten der Formel I, dadurch gekennzeichnet, daß man eine Verbindung der Formel II

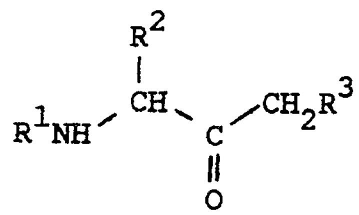

II

mit einem Trialkylchlorsilan in eine Verbindung der Formel III

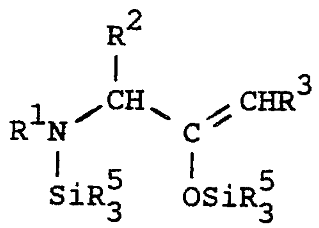

III

überführt, dann die Verbindung der Formel III mit einem Diazoessigester der Formel $N_2CH_2COOR^4$ ($R^4 \neq H$) zu einer Cyclopropanverbindung der Formel IV

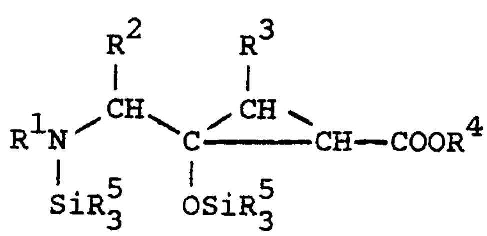

IV

umsetzt, und anschließend die Verbindung der Formel IV durch Ringöffnung und Abspaltung der Silylgruppen sowie gegebenenfalls Verseifung in die Verbindung der Formel I überführt, wobei die bereits Gruppen $R^1$, $R^2$, $R^3$ und $R^4$ in den Formeln II-IV die bereits angegebenen Bedeutungen haben und $R^5$ eine Alkylgruppe mit 1 bis 5 C-Atomen darstellt.

Vor- und nachstehend haben die Reste $R^1$, $R^2$, $R^3$, $R^4$ und $R^5$ die angegebenen Bedeutungen, falls nicht ausdrücklich etwas anderen angegeben ist.

Erfindungsgemäß kann Alkyl oder Alkylen mit 1-5 C-Atomen unverzweigt oder verzweigt sein. Vorzugsweise bedeutet Alkyl Methyl, Ethyl, Propyl, Butyl oder Pentyl, aber auch Isopropyl, Isobutyl, sek.- oder tert.-Butyl, 3-Methylbutyl, 1,1-, 1,2- oder 2,2-Dimethylpropyl oder 1-Ethylpropyl. Vorzugsweise bedeutet Alkylen Methylen, Ethylen, Propylen, ferner auch Butylen oder Pentylen.

$R^1$ in den Formeln I-IV bedeut eine den Reaktionsablauf nicht störende Aminoschutzgruppe oder nach Abspaltung dieser Gruppe auch H.

Der Ausdruck "Aminoschutzgruppe" ist allgemein bekannt und bezieht sich auf Gruppen, die geeignet sind, eine Aminogruppe vor chemischen Umsetzungen zu schützen (zu blockieren), die aber leicht entfernbar sind, nachdem die gewünschte chemische Reaktion an einer anderen Stelle des Moleküls durchgeführt worden ist. Typisch für solche Gruppen sind insbesondere unsubstituierte oder substituierte Acyl-, Aryl- (z.B. 2,4-Dinitrophenyl DNP), Aralkoxymethyl- (z.B. Benzyloxymethyl BOM) oder Aralkylgruppen (z.B. Benzyl, 4-Nitrobenzyl, Triphenylmethyl). Da die Aminoschutzgruppen nach der gewünschten Reaktion (oder Reaktionsfolge) entfernt werden, ist ihre Art und Größe im übrigen nicht kritisch; bevorzugt werden jedoch solche mit 1-20, insbesondere 1-8 C-Atomen. Der Ausdruck "Acylgruppe" ist im Zusammenhang mit dem vorliegenden Verfahren im weitesten Sinne aufzufassen. Er umschließt von aliphatischen, araliphatischen, aromatischen oder heterocyclischen Carbonsäuren oder Sulfonsäuren abgeleitete Acylgruppen sowie insbesondere Alkoxycarbonyl-, Aryloxycarbonyl- und vor allem Aralkoxycarbonylgruppen. Beispiele

für derartige Acylgruppen sind Alkanoyl wie Acetyl, Propionyl, Butyryl; Aralkanoyl wie Phenylacetyl; Aroyl wie Benzoyl oder Toluyl; Aryloxyalkanoyl wie Phenoxyacetyl; Alkoxycarbonyl wie Methoxycarbonyl, Ethoxycarbonyl (ETOC), 2,2,2-Trichlorethoxycarbonyl, Isopropoxycarbonyl (IPOC), tert.-Butoxycarbonyl (BOC), 2-Jodethoxycarbonyl; Aralkyl oxycarbonyl wie Benzyloxycarbonyl (CBZ), 4-Methoxybenzyloxycarbonyl, 9-Fluorenylmethoxycarbonyl (FMOC). Bevorzugte Aminoschutzgruppen sind BOC, DNP und BOM, ferner CBZ, FMOC, Benzyl und Acetyl.

$R^2$ bedeutet vorzugsweise eine Alkylgruppe mit den dafür bereits angegebenen bevorzugten Bedeutungen.

Bevorzugt für $R^2$ sind auch Alkylgruppen, die ein- oder zweifach substituiert sind durch -OH, -COOH, $-CONH_2$ oder $-NH_2$.

Demnach sind auch folgende Reste $R^2$ besonders bevorzugt: $-CH_2OH$, $-CH(OH)-CH_3$, $-CH_2-COOH$, $-CH_2-CONH_2$, $-CH_2-CH_2-COOH$, $-CH_2-CH_2-CONH_2$, $-CH_2-CH_2-CH_2-NH_2$, $-CH_2-CH_2-CH_2-CH_2-NH_2$.

In den Alkylgruppen für $R^2$ kann auch eine $CH_2$-Gruppe durch -S- ersetzt sein.

Weiterhin sind unsubstituierte oder substituierte Phenyl-, Phenylalkyl-, Cyclohexyl- oder Cyclohexylalkylgruppen für $R^2$ bevorzugt. Diese Gruppen können ein- oder mehrfach in 2-, 3- und/oder 4-Stellung substituiert sein. Bevorzugt ist eine Einfachsubstitution in 4-Stellung. Bevorzugte Substituenten im Falle von substituiertem Phenyl oder Phenylalkyl sind Fluor, Chlor, Hydroxy, Alkyl oder Alkoxy mit 1 bis 5 C-Atomen. Im Falle von substituiertem Cyclohexyl oder Cyclohexylakyl sind als Substiuenten Alkylgruppen bevorzugt.

Phenylalkyl oder Cyclohexylalkyl bedeuten vorzugsweise Phenyl- oder Cyclohexylmethyl, Phenyl- oder Cyclohexylethyl oder auch Phenyl- oder Cyclohexylpropyl. Demnach sind folgende Gruppen bevorzugt: Benzyl (Phenylmethyl), Cyclohexylmethyl, 1- oder 2-Phenylethyl, 1- oder 2-Cyclohexylethyl, o-, m- oder p-Methylbenzyl, 1- oder 2-o-, -m- oder -p-Tolylethyl, o-, m- oder p-Ethylbenzyl, 1- oder 2-o-, -m- oder -p-Ethylphenylethyl, o-, m- oder p-Methoxybenzyl, 1- oder 2-o-, -m- oder -p-Methoxyphenylethyl, o-, m- oder p-Fluorbenzyl, 1- oder 2-o-, -m- oder -p-Fluorphenylethyl, o-, m- oder p-Chlorbenzyl, 1- oder 2-o-, -m-oder -p-Chlorphenylethyl, o- m- oder p-Brombenzyl, 1- oder 2-o-, -m- oder -p-Bromphenylethyl, o-, m- oder p-Jodbenzyl, 1- oder 2-o-, -m- oder -p-Jodphenylethyl, o-, m- oder p-Hydroxybenzyl, 2,3-, 2,4-, 2,5-, 2,6-, 3,4- oder 3,5-Dimethoxybenzyl, 3,4,5-Trimethoxybenzyl, o-, m- oder p-Aminobenzyl, 2-, 3- oder 4-Methylcyclohexylmethyl, 2-, 3- oder 4-Ethylcyclohexylmethyl, 2-, 3- oder 4-tert. Butylcyclohexylmethyl oder 1- oder 2-(2-, (3- oder (4-Ethylcyclohexyl)-ethyl

$R^2$ ist auch bevorzugt Het-alkyl-.

Het ist vorzugsweise 1-, 2- oder 3-Pyrrolyl, 1-, 2-, 3-, 4-, 5-, 6- oder 7-Indolyl, 1-, 2-, 3-, 4-, 5-, 6-, 7-Isoindolyl, 1-, 3-, 4- oder 5-Pyrazolyl, 1-, 2-, 4- oder 5-Imidazolyl, 1-, 2-, 4- oder 5-Benzimidazolyl, 2-, 3-oder 4-Pyridyl, 2-, 4-, 5- oder 6-Pyrimidinyl, weiterhin bevorzugt 3- oder 4-Pyridazinyl oder Pyrazinyl, Chinolyl oder Isochinolyl. Die heterocyclischen Reste können auch teilweise oder vollständig hydriert sein und demnach beispielsweise 2,3-Dihydropyrrolyl, 2,5-Dihydropyrrolyl, Pyrrolidinyl, Tetrahydroimidazolyl, 2,3-Dihydropyrazolyl, Tetrahydropyrazolyl, 1,4-Dihydropyridyl, 1,2,3,4-Tetrahydropyridyl, 1,2,3,6-Tetrahydropyridyl oder Piperidinyl bedeuten.

Falls in $R^2$ funktionelle Gruppen enthalten sind, werden diese vorzugsweise vor Durchführung der enantioselektiven Synthese mit entsprechenden, literaturbekannten Schutzgruppen geschützt, die dann anschließend wieder abgespalten werden.

Es können also auch mehrere - gleiche oder verschiedene -geschützte Amino- und/oder Hydroxygruppen im Molekül des Ausgangsstoffes vorhanden sein. Falls die vorhandenen Schutzgruppen voneinander verschieden sind, können sie in vielen Fällen selektiv abgespalten werden.

Die Reste $R^3$ und $R^4$ bedeuten H oder Alkyl mit 1-5 C-Atomen. Für die Alkylgruppen können dabei die bereits definierten, bevorzugten Gruppen in Betracht.

$R^5$ in der Verbindung Trialkylchlorsilan bedeutet eine Alkylgruppe mit 1-5 C-Atomen mit den für Alkyl bereits angegebenen bevorzugten Bedeutungen. Insbesondere bevorzugt sind die Bedeutungen Methyl oder Ethyl.

Als Ausgangsprodukt wird das gewünschte (R)- oder (S)-Enantiomer eingesetzt, und man erhält dann unter Erhalt der Konfiguration das entsprechende Produkt der Formel I in der (R)- bzw. (S)-Form. Die Ausgangsprodukte, die Aminoketone der Formel II, sind zugänglich nach bekannten Herstellungsmethoden, beschrieben z.B. in Chem. Pharm. Bull. 36 , 3341 (1988) von S. Kano et al.

Der erste Schritt der Synthese, die Darstellung der Silylenolether der Formel III aus den Aminoketonen II, erfolgt durch Umsetzung mit einem Trialkylchlorsilan in Gegenwart einer starken, organischen Base, wie z.B. eines Metallamids, Lithium- oder Magnesiumdialkylamids, insbesondere Lithiumdiisopropylamid, nach Standardmethoden. Als Lösungsmittel eignen sich beispielsweise Tetrahydrofuran, Dialkylether, Mischungen aus Tetrahydrofuran mit Alkanen, wie z.B. Pentan oder Hexan, oder Mischungen verschiedener Ether.

Die Reaktionstemperaturen liegen vorzugsweise zwischen -120° und -40°, insbesondere zwischen

-80° und -60°.

Vorzugsweise läßt man nach beendeter Reaktion bei diesen tiefen Temperaturen (die Reaktionsdauer kann zwischen 30 Minuten und 8-10 Stunden liegen) das Reaktionsgemisch innerhalb von einigen Stunden langsam auf Normaltemperatur kommen. Die Aufarbeitung erfolgt dann nach Standardmethoden.

Der zweite Schritt, die Herstellung der Cyclopropanderivate der Formel IV, erfolgt durch Addition eines Diazoessigsäureesters der Formel $N_2CH_2COOR^4$, worin $R^4$ Alkyl mit 1-5 C-Atomen, hier also nicht H bedeutet, an die Doppelbindung der Verbindung der Formel III in Gegenwart einer Kupferverbindung, vorzugsweise eines Kupfersalzes oder Kupferkomplexes. Als beispielhafte Vertreter solcher Kupferverbindungen seien Kupferacetat, Kupferhalogenide, Bis-(salicylaldiminato)-kupfer-komplex, Bis-(acetylacetonato)-kupfer(II)-komplex genannt. Vorzugsweise wird der Bis-(acetylacetonato)-kupfer(II)-komplex eingesetzt. Ansonsten wird diese Cycloaddition nach Standardmethoden durchgeführt, wie sie in den Lehrbüchern der organischen Chemie beschrieben sind.

Man setzt vorzugsweise ein inertes aromatisches Lösungsmittel ein, z.B. Benzol, Toluol oder Xylol, und erhitzt unter Rückfluß vorzugsweise auf 70°-100°, insbesondere bevorzugt auf 80°, bis zur Beendigung der Stickstoffentwicklung.

Diese beiden Synthesestufen werden vorzugsweise unter Schutzgasatmosphäre durchgeführt.

Der letzte Syntheseschritt, die Ringöffnung und gleichzeitige Abspaltung der Silylgruppen erfolgt durch Umsetzung der Cyclopropanderivate der Formel IV in einem inerten Lösungsmittel mit einem Fluorsalz. Als Lösungsmittel sind Halogenkohlenwasserstoffe, wie z.B. Dichlormethan, Tetrachlormethan oder auch Kohlenwasserstoffe oder Ether geeignet.

Als Fluorsalze eignen sich z.B. Metallfluoride wie NaF oder KF, Ammoniumfluoride wie z.B. $BU_4NF$ oder auch HF in Form von $NEt_3 \cdot HF$.

Die Reaktionsdurchführung erfolgt vorzugsweise bei Temperaturen zwischen 10° und 100°, insbesondere bevorzugt zwischen 15° und 30°. Die Reaktionsdauer liegt je nach Ansatzgröße zwischen 30 Minuten und 10 Stunden. Die Aufarbeitung erfolgt zweckmäßig durch Extraktion.

Erfindungsgemäß werden nach üblicher einfacher Aufarbeitungs- und Aufreinigungsmethodik beispielsweise mittels Chromatographie und/oder Kristallisation die $\gamma$-Keto-$\delta$-Aminosäurederivate der Formel I erhalten.

Die Aminoschutzgruppe $R^1$ kann dann, falls gewünscht, nach literaturbekannten Methoden abgespalten werden, dann bedeutet $R^1$ = H. Gegebenenfalls kann die Estergruppe $-COOR^4$ noch verseift werden, dann bedeutet in Formel I $R^4$ = H.

Mit dem erfindungsgemäßen Verfahren können nun in hoher Stereoselektivität und mit guten Ausbeuten diese wertvollen Ketomethylenanaloge eines Dipeptids hergestellt werden.

Beispielsweise können mit dem erfindungsgemäßen Verfahren die Ketomethylenanaloge folgender Dipeptide hergestellt werden:

Phe-Gly, Ala-Gly, Val-Gly, Leu-Gly, Ile-Gly, Ser-Gly, Thr-Gly, Asx-Gly, Lys-Gly, His-Gly, Met-Gly, Tyr-Gly, Trp-Gly oder auch Orn-Gly.

Die Abkürzungen haben folgende Bedeutung:

Phe = Phenylalanin
Gly = Glycin
Ala = Alanin
Val = Valin
Leu = Leucin
Ile = Isoleucin
Ser = Serin
Thr = Threonin
Asx = Asparagin oder Asparaginsäure
Lys = Lysin
His = Histidin
Met = Methionin
Tyr = Tyrosin
Trp = Tryptophan
und
Orn = Ornithin

Die folgenden Beispiele sollen das erfindungsgemäße Verfahren näher beschreiben, ohne es zu begrenzen. Die Temperaturangabe erfolgt immer in Grad Celsius. Fp. bedeutet Schmelzpunkt.

Beispiel 1

a) 2,00 g (7,60 mmol) 1-(N-tert.-Butyloxycarbonylamino)-2-(S)-phenylethylmethylketon (Herstellung analog S. Kano et al., Chem.Pharm.Bull 36 , 3341 (1988)) werden in 30 ml TBF zu einer Lithiumdiisopropylamidlösung (hergestellt bei -78° aus 1,93 g (19,00 mmol) Diisopropylamin und 7,6 ml (19,00 mmol) n-Butyllithium in Hexan (2,5 M) unter Schutzgasatmosphäre) bei -78° gegeben. Man rührt ca. 2 Stunden bei -78° und gibt dann 2,47 g (22,80 mol) Trimethylchlorsilan zu, läßt über Nacht auf Raumtemperatur kommen, gibt wenig trockenes Triethylamin zu und entfernt das Lösungsmittel. Zum Rückstand gibt man 20 ml Pentan, filtriert den ausgefallenen Feststoff ab, engt das Filtrat ein und begast mit Stickstoff. Durch Reinigung durch eine Kugelrohr-Destillation erhält man bei 120-140°/0,2 Torr den entsprechenden Silylenolether.

b) 2,89 g (7,1 mmol) des in a) erhaltenen Produktes werden mit 5 ml Benzol und 47 mg (0,18 mmol) Bis-(acetylacetonato)-kupfer(II)-komplex (Cu (acac)$_2$) unter $N_2$-Atmosphäre vorgelegt. Dazu gibt man unter Erwärmen auf 90° innerhalb von 3 Stunden 1,40 g (14 mmol) Diazoessigsäuremethylester in 15 ml Benzol. Nach beendeter Stickstoffentwicklung wird das Lösungsmittel entfernt, der Rückstand mit 0,5 g Aluminiumoxid (neutral, Aktivitätsstufe III) versetzt und durch eine kurze Säule mit 7 g Aluminiumoxid filtriert. Die Elution erfolgt mit Pentan. Zur weiteren Reinigung wird das Rohprodukt nochmals durch 10 g $Al_2O_3$ filtriert. Eluiert wird mit Pentan und anschließend mit Methyl-tert.-butylether, woraus das entsprechende (S)-Cyclopropanderivat isoliert wird.

c) Ein Gemisch aus 1,38 g (2,5 mmol) des in b) hergestellten Cyclopropanderivates, 20 ml $CH_2Cl_2$ und 0,44 g (2,5 mmol) $NEt_3$ • HF wird ca. 1 Stunde lang gerührt. Man gibt dann 15 ml $H_2O$ zu und arbeitet extraktiv mit $CH_2Cl_2$ auf. Das Rohprodukt wird durch Säulenchromatographie an Kieselgel (Elution mit Pentan/Essigester = 4/1) und anschließende Kristallisation aus Pentan unter Zusatz von wenig Methyl-tert.-butylether gereinigt. Man erhält spektroskopisch reinen (S)-Ketoester, der dem Ketomethylen-analogen von (S)-Phe-Gly entspricht, mit Fp. = 58-59°.

Der vollständige Reaktionsablauf ist in Schema 1 dargestellt:

Schema 1

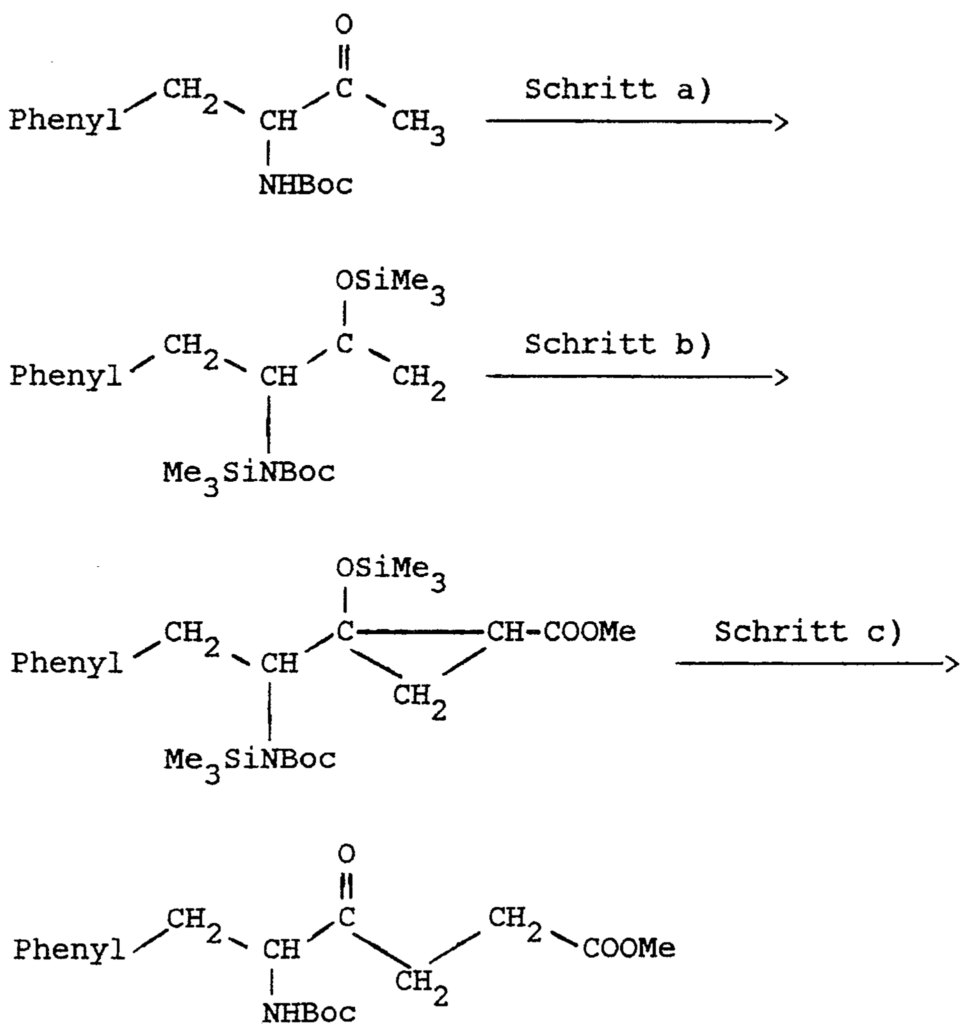


## Ansprüche

1. Verfahren zur enantioselektiven Herstellung von γ-Keto-δ-Aminosäurederivaten der Formel I

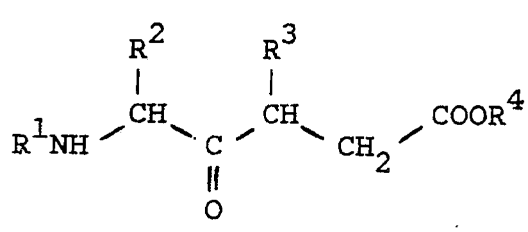

I

worin
$R^1$ eine Aminoschutzgruppe oder H,
$R^2$ eine Alkylgruppe mit 1 bis 5 C-Atomen, die gegebenenfalls ein- oder zweifach substituiert sein kann durch -OH, -COOH, $-CONH_2$ oder $-NH_2$ oder worin gegebenenfalls eine $CH_2$-Gruppe durch -S- ersetzt sein kann, unsubstituiertes oder durch 1 bis 5 C-Atome enthaltendes Alkyl oder Alkoxy, Halogen, Hydroxy oder Amino ein-oder mehrfach substituiertes Phenyl oder Cyclohexyl, Phenylalkyl oder Cyclohexylalkyl oder Het-alkyl-, wobei
Het einen gesättigten oder ungesättigten 5- oder 6-gliedrigen heterocyclischen Rest mit 1-4 N-Atomen, der mit einem Benzolring kondensiert sein kann, und
-alkyl- eine Alkylengruppe mit 1-5 C-Atomen darstellt,
und
$R^3$ und $R^4$ jeweils unabhängig voneinander H oder Alkyl mit 1-5 C-Atomen
bedeuten,
dadurch gekennzeichnet, daß man eine Verbindung der Formel II

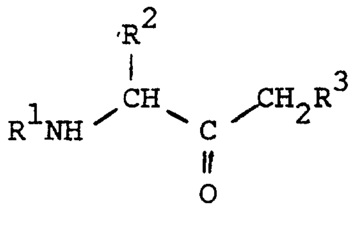

II

mit einem Trialkylchlorsilan in eine Verbindung der Formel III

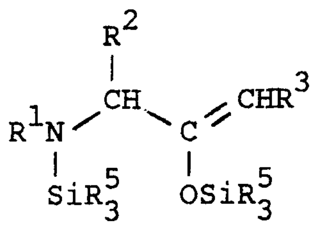

III

überführt, dann die Verbindung der Formel III mit einem Diazoessigester der Formel $N_2CH_2COOR^4$ ($R^4 \neq H$) zu einer Cyclopropanverbindung der Formel IV

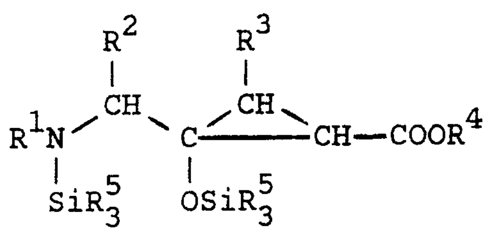

IV

umsetzt, und anschließend die Verbindung der Formel IV durch Ringöffnung und Abspaltung der Silylgruppen sowie gegebenenfalls Verseifung in die Verbindung der Formel I überführt, wobei die Gruppen $R^1$, $R^2$, $R^3$ und $R^4$ in den Formeln II-IV die bereits angegebenen Bedeutungen haben und $R^5$ eine Alkylgruppe mit 1 bis 5 C-Atomen darstellt.